# EUROPÄISCHE PATENTSCHRIFT

(11) **EP 0 379 862 B1**
(45) Veröffentlichungstag und Bekanntmachung des Hinweises auf die Patenterteilung: **17.03.1993**
(21) Anmeldenummer: 90100126.3
(22) Anmeldetag: 04.01.1990
(51) Int. Cl.: C07C 37/84, C07C 39/16

(54) **Verfahren zur Reinigung von rohem 2,2-Bis-(3,5-dimethyl-4-hydroxphenyl)-propan**
Method for the purification of crude 2,2-bis-(3,5-dimethyl-4-hydroxyphenyl)-propane
Procédé pour l'épuration de propane-2,2-bis-(phényl-hydroxy-4-diméthyl-3,5) brut

(30) Priorität: 12.01.1989 DE 3900680
(43) Veröffentlichungstag der Anmeldung: 01.08.1990
(73) Patentinhaber: BAYER AG, 51368 Leverkusen (DE)
(72) Erfinder: Rauchschwalbe, Günter, D-5090 Leverkusen (DE); Blank, Heinz-Ulrich, Dr., D-5068 Odenthal (DE); Handschuh, Volkmar, Dr., D-5093 Burscheid (DE); Griehsel, Bernd, Dr., D-4250 Bottrop (DE)

(56) Entgegenhaltungen:
- DE-A- 2 749 278
- DE-A- 2 758 565
- DE-A- 2 758 566
- DE-A- 2 853 883
- GB-A- 1 323 066

## Beschreibung

Die vorliegende Erfindung betrifft ein Verfahren zur Reinigung von rohem 2,2-Bis-(3,5-dimethyl-4-hydroxy-phenyl)-propan (= Tetramethyl-bisphenol A = TMBPA) durch Umkristallisation aus wasserhaltigen Alkoholen.

Es ist prinzipiell bekannt, Bisphenole aus wasserhaltigen niederen Alkoholen umzukristallisieren. DE-OS 27 58 565 beschreibt hierzu in der Hauptsache Glykole oder deren Ether, sowie Glyzerin, Phenol und Ethanolamin als zu verwendende alkoholische Lösungsmittel. Diese Lösungsmittel sind jedoch nur schwierig wiederzugewinnen und dürfen wegen ihrer Toxizität (z.B. Fischtoxizität) nicht in das Abwasser gelangen, sondern bedürfen einer separaten Entsorgung. Die genannte DE-OS führt am Beispiel von umzukristallisierendem Bisphenol A auch ein Lösungsmittelgemisch aus Ethanol und Wasser an. Hierbei werden für 50 g Bisphenol A eine Menge von 500 g Wasser und 102 g Ethanol eingesetzt, um das Bisphenol A bei 100°C vollständig aufzulösen. Für 50 g des umzukristallisierenden Bisphenols A werden also mehr als 600 g gesamtes Lösungsmittel benötigt. Dem Reinigungsverfahren der obigen DE-OS liegt offenbar der Gedanke zugrunde, daß vorwiegend ein schlechtes Lösungsmittel mit einem hohen Gehalt an Wasser eingesetzt werden muß, um die Kristallisationsverluste gering zu halten.

TMBPA kann unter diesen Bedingungen jedoch nicht umkristallisiert werden.

Es wurde nun gefunden, daß man im Falle von TMBPA, entgegen der beschriebenen Lehre, das Verhältnis von Alkohol zu Wasser stark erhöhen kann und gleichzeitig das Verhältnis des wäßrigen Alkohols zum TMBPA erheblich senken kann, wobei die Raumausbeute dieses Verfahrens erheblich gesteigert werden kann und dennoch hohe Rückgewinnungsraten an umkristallisiertem reinen TMBPA erzielt werden. Dies war bei der höheren Lösefähigkeit eines solchen Alkohol/Wasser-Gemisches nicht zu erwarten. Eine geringere Rückgewinnungsrate würde zwangsläufig auch mit einer erhöhten Belastung der entstehenden Abwässer einhergehen. Beide Effekte würden einem wirtschaftlichen Verfahren im Wege stehen.

So wurde überraschenderweise gefunden, daß beispielsweise ein Gemisch aus 30 g Wasser und 120 g Methanol bei 100°C wenigstens 95 g reines TMBPA auflöst und beim Abkühlen 92 % des eingesetzten TMBPA wiedergewonnen werden. Das ist, bezogen auf die eingesetzte Menge an Substrat, weniger als ein Viertel des gesamten Lösungsmittelgemisches, welches gemäß der genannten DE-OS benötigt wird, und gleichzeitig nur etwa 60 % der Menge des alkoholischen Lösungsmittels dieser DE-OS. Gleichzeitig ist das wiedergewonnene Substrat sehr viel reiner als das in der obigen DE-OS beschriebene. So wird gemäß dieser DE-OS eine Anreicherung von 93,1 % auf 96,0 % am gewünschten p,p′-Isomeren erreicht, während im vorliegenden Verfahren beispielsweise eine Anreicherung von 93 bis 95 % auf 99,7 % erreicht wird. Solche Ergebnisse waren angesichts des bekannten Standes der Technik nicht zu erwarten.

Die Erfindung betrifft demnach ein Verfahren zur Reinigung von rohem 2,2-Bis-(3,5-dimethyl-4-hydroxyphenyl)propan (= Tetramethyl-bisphenol A = TMBPA) durch Umkristallisation aus wasserhaltigen Alkoholen, das dadurch gekennzeichnet ist, daß man einen C₁-C₄-Monoalkohol mit einem Gehalt von 2 bis 40 Gew.-% Wasser, bezogen auf das Gesamtgewicht von Alkohol und Wasser, in einer Menge von 50 bis 250 Gew.-%, bezogen auf die Menge an TMBPA, einsetzt und bei einer Temperatur von 65 bis 150°C arbeitet.

Alkohole für das erfindungsgemäße Verfahren sind Methanol, Ethanol, Propanol, Isopropanol, Butanol, Isobutanol oder tert.-Butanol, bevorzugt ein C₁-C₃-Monoalkohol, wie Methanol, Ethanol, Propanol oder Isopropanol, besonders bevorzugt Methanol oder Ethanol und ganz besonders bevorzugt Methanol.

Die gesamte Lösungsmittelmenge enthält 2 bis 40 Gew.-% Wasser, bevorzugt 5 bis 40 Gew.-%, besonders bevorzugt 10 bis 30 Gew.-% und ganz besonders bevorzugt 15 bis 25 Gew.-% Wasser. Dieses Lösungsmittelgemisch wird in einer Menge von 50 bis 250 Gew.-%, bezogen auf die Menge des umzukristallisierenden TMBPA, bevorzugt in einer Menge von 50 bis 200 Gew.-%, besonders bevorzugt von 80 bis 150 Gew.-%, eingesetzt.

Die erfindungsgemäße Umkristallisation wird bei einer Temperatur von 65 bis 150°C, bevorzugt bei 80 bis 120°C, durchgeführt. Soweit der Siedepunkt des eingesetzten Monoalkohols überschritten wird, wird bei erhöhtem Druck gearbeitet. Als Druck kommt demzufolge ein solcher von 1 bis 20 bar, bevorzugt 1,2 bis 5 bar, infrage. Dieser Druck kann der Eigendruck des gesamten Umkristallisationssystems oder ein Fremddruck, beispielsweise durch aufgedrückten Stickstoff. In bevorzugter Weisse wird unter dem sich einstellenden Eigendruck gearbeitet. In vielen Fällen kann es günstig sein, den hierzu erforderlichen Druckbehälter mit dem gesamten Einsatz an umzukristallisierendem TMBPA und dem Lösungsmittelgemisch zu füllen, bis (nahe) zum Sieden zu erhitzen und erst dann druckdicht zu verschließen. Dadurch wird der Gesamtdruck durch Wegfall des Partialdruckes von Luft erniedrigt.

Eine weitere vorteilhafte Arbeitsweise besteht darin, den Alkoholteil des Lösungsmittelgemisches vorzulegen und das TMBPA darin zu suspendieren. Sodann wird zum Sieden erhitzt und erst dann der Wasseranteil zugegeben, je nach den gewählten Mischungsverhältnissen kann bereits an diesem Punkt reines TMBPA ausfallen; in jedem Falle wird aber das gesamte Umkristallisationssystem bis auf Raumtemperatur unter Rühren abgekühlt. Je nach der Qualität des rohen TMBPA kann es weiterhin vorteilhaft sein, die heiße Lösung von TMBPA mit Klärhilfsmitteln, wie Aktivkohle, Kieselgur, Kieselgel, Zellulosepulver u.a. zu behandeln, diese Klärhilfsmittel abzufiltrieren und erst dann unter Rühren abzukühlen. Hierbei kann der Zusatz von Wasser vor oder nach einer solchen Klärfiltration erfolgen.

Weiterhin vorteilhaft kann ein Zusatz von löslichen reduzierend wirkenden Stoffen sein. Solche Stoffe sind beispielsweise Sulfite, Hydrogensulfite, Dithionite oder Boranate von Alkalimetallen oder Erdalkalimetallen, bevorzugt von Natrium. Als weiteres Reduktionsmittel sei Rongalit genannt. Solche Reduktionszusätze werden in einer Menge von 0,1 bis 5 Gew.-%, bevorzugt 0,5 bis 2 Gew.-%, bezogen auf TMBPA, zugesetzt. In bevorzugter Weise werden Natriumdithionit oder Rongalit zugesetzt.

Das im erfindungsgemäßen Verfahren erhaltene TMBPA ist reinweiß, gut filtrierbar und von hohe Reinheit. TMBPA ist Ausgangsmaterial für die Herstellung von thermoplastischen Chemiewerkstoffen.

### Beispiel 1

In einem Autoklaven erwärmte man unter Rühren eine Mischung aus 600 ml (480 g) Methanol, 120 ml Wasser, 1 g Na₂S₂O₄ und 400 g technischem (ca. 95 %iges) rot verfärbtem TMBPA auf etwa 100°C. Dabei stellte sich ein Druck von etwa 2 bar ein. Man kühlte unter Rühren ab und filtrierte. Der Filterkuchen wurde zunächst mit 50%igem Methanol, dann mit Wasser gewaschen und getrocknet. Man erhielt 350 g reinweißes TMBPA mit einem Gehalt von 99,7 Gew.-% laut gaschromatographischer Analyse und einem Fp. von 166,5 bis 167,5°C. Das stellte eine Wiedergewinnungsrate von 92 % des im Einsatzmaterial vorhandenen TMBPA dar. Die Mutterlauge wurde zur Wiedergewinnung des Lösungsmittel destillativ aufgearbeitet. Der Rückstand (Suspension in Wasser) wurde abfiltriert. Der feste Rückstand wurde durch Verbrennen entsorgt; der wäßrige Ablauf war biologisch gut abbaubar.

### Beispiel 2

In einem Glasautoklaven erwärmte man eine Mischung aus 250 g 95%igem TMBPA und 250 g 70%igem Methanol (175 g MeOH + 75 g H₂O) sowie 1,2 g Na₂S₂O₄ bis auf etwa 100°C. Dabei ging das TMBPA vollständig in Lösung. Es stellte sich ein Druck von etwa 1,9 bar ein. Man kühlte auf Raumtemperatur ab. Nach Aufarbeitung (wie in Beispiel 1) erhielt man 227 g weißes TMBPA (99,6%ig); das entspricht einer Wiedergewinnungsrate von 95 %; die Raumausbeute erreicht ca. 420 g/l.

### Beispiel 3

In einem Glasautoklaven erwärmte man eine Mischung aus 250 g 95%igem TMBPA und 250 g 90%igem Ethanol (225 g EtOH + 25 g H₂O) sowie 1,2 g Na₂S₂O₄ bis auf 97°C. Dabei ging das TMBPA vollständig in Lösung. Es stellte sich ein Druck von 1,4 bar ein. Man kühlte auf Raumtemperatur ab. Nach Aufarbeitung (wie in Beispiel 1) erhielt man 191 g weißes TMBPA (99,8%ig); das entspricht einer Wiedergewinnungsrate von 80%.

### Beispiel 4

In einem Glasautoklaven erwärmte man eine Mischung aus 300 g 95%igem TMBPA (-̂ 285 g/100 %), 285 g 70%igem 2-Propanol (200 g 2-Propanol + 85 g H₂O) und 1,2 g Na₂S₂O₄ bis auf 102°C. Dabei ging das TMBPA vollständig in Lösung; es stellte sich ein Druck von 1,3 bar ein. Man kühlte ab und isolierte (nach Aufarbeiten wie in Beispiel 1) 263 g 99,5 %iges TMBPA. Das entspricht einer Wiedergewinnungsrate von 92 %.

### Beispiel 5

Man erwärmte in einem unverschlossenen Gefäß 200 g Methanol bis zum Sieden am Rückfluß, löste darin 140 g reines TMBPA, das dabei gerade völlig gelöst wurde, und tropfte zu der siedenden Lösung 40 g Wasser. Man kühlte auf 25°C auf und isolierte 125 g TMBPA (Wiedergewinnungsrate 89 %).

## Patentansprüche

1. Verfahren zur Reinigung von rohem 2,2-Bis-(3,5-dimethyl-4-hydroxyphenyl)-propan (= Tetramethylbisphenol A = TMBPA) durch Umkristallisation aus wasserhaltigen Alkoholen, dadurch gekennzeichnet, daß man einen C₁-C₄-Monoalkohol mit einem Gehalt von 2 bis 40 Gew.-% Wasser, bezogen auf das Gesamtgewicht von Alkohol und Wasser, in einer Menge von 50 bis 250 Gew.-%, bezogen auf die Menge an TMBPA, einsetzt und bei einer Temperatur von 65 bis 150°C arbeitet.

2. Verfahren nach Anspruch 1, dadurch gekennzeichnet, daß man einen C₁-C₃-Monoalkohol, bevorzugt Methanol oder Ethanol, besonders bevorzugt Methanol, einsetzt.

3. Verfahren nach Anspruch 1, dadurch gekennzeichnet, daß die gesamte Lösungsmittelmenge 5 bis 40 Gew.-%, bevorzugt 10 bis 30 Gew.-%, besonders bevorzugt 15 bis 25 Gew.-%, Wasser enthält.

4. Verfahren nach Anspruch 1, dadurch gekennzeichnet, daß das Lösungsmittelgemisch in einer Menge von 50 bis 200 Gew.%, bevorzugt 80 bis 150 Gew.-%, bezogen auf das umzukristallisierende TMBPA, eingesetzt wird.

5. Verfahren nach Anspruch 1, dadurch gekennzeichnet, daß bei einer Temperatur von 80 bis 120°C gearbeitet wird.

6. Verfahren nach Anspruch 5, dadurch gekennzeichnet, daß bei einem Druck von 1 bis 10 bar, bevorzugt 1,2 bis 5 bar, besonders bevorzugt beim Eigendruck des Systems, gearbeitet wird.

7. Verfahren nach Anspruch 1, dadurch gekennzeichnet, daß in Gegenwart von löslichen, reduzierenden Zusatzstoffen in einer Menge von 0,1 bis 5 Gew.-%, bezogen auf TMBPA, gearbeitet wird.

8. Verfahren nach Anspruch 7, dadurch gekennzeichnet, daß in Gegenwart von Natriumdithionit oder Rongalit gearbeitet wird.

## Claims

1. Process for the purification of crude 2,2-bis-(3,5-dimethyl-4-hydroxyphenyl)-propane (= tetramethylbisphenol A = TMBPA) by recrystallization from aqueous solutions of alcohols, characterized in that a C₁-C₄-monoalcohol with a water content of 2 to 40% by weight relative to the total weight of alcohol and water is used in an amount of from 50 to 250% by weight relative to the amount of TMBPA, and the operation is carried out at a temperature of 65 to 150°C.

2. Process according to Claim 1, characterized in that a C₁-C₃-monoalcohol, preferably methanol or ethanol, particularly preferably methanol, is used.

3. Process according to Claim 1, characterized in that the total amount of solvent contains 5 to 40% by weight, preferably 10 to 30% by weight, particularly preferably 15 to 25% by weight of water.

4. Process according to Claim 1, characterized in that the solvent mixture is used in an amount of from 50 to 200% by weight, preferably in an amount from 80 to 150% by weight relative to the TMBPA which is to be recrystallized.

5. Process according to Claim 1, characterized in that it is operated at a temperature of 80 to 120°C.

6. Process according to Claim 5, characterized in that it is operated at a pressure of 1 to 10 bar, preferably 1.2 to 5 bar, particularly preferably at the autogenous pressure of the system.

7. Process according to Claim 1, characterized in that it is operated in the presence of soluble, reducing agent additives in an amount of from 0.1 to 5% by weight, relative to TMBPA.

8. Process according to Claim 7, characterized in that it is operated in the presence of sodium dithionite or rongalite.

## Revendications

1. Procédé pour purifier le 2,2-bis-(3,5-diméthyl-4-hydroxyphényl)propane brut (= tétraméthyl-bis-phénol A = TMBPA) par recristallisation dans des alcools aqueux, caractérisé en ce que l'on utilise un monoalcool en C₁-C₄ à une teneur de 2 à 40 % en poids d'eau par rapport au poids total d'alcool et d'eau, en quantité de 50 à 250 % du poids du TMBPA, et on opère à une température de 65 à 150°C.

2. Procédé selon la revendication 1, caractérisé en ce que l'on utilise un monoalcool en C₁-C₃, de préférence le méthanol ou l'éthanol, tout spécialement le méthanol.

3. Procédé selon la revendication 1, caractérisé en ce que la quantité totale de solvant contient de 5 à 40 % en poids, de préférence de 10 à 30 % en poids et plus spécialement de 15 à 25 % en poids d'eau.

4. Procédé selon la revendication 1, caractérisé en ce que le mélange solvant est mis en oeuvre en quantité de 50 à 200 %, de préférence de 80 à 150 % du poids du TMBPA à recristalliser.

5. Procédé selon la revendication 1, caractérisé en ce que l'on opère à une température de 80 à 120°C.

6. Procédé selon la revendication 5, caractérisé en ce que l'on opère sous une pression de 1 à 10 bar, de préférence de 1,2 à 5 bar, et tout spécialement sous la pression propre du système.

7. Procédé selon la revendication 1, caractérisé en ce que l'on opère en présence d'additifs réducteurs solubles en quantité de 0,1 à 5 % du poids du TMBPA.

8. Procédé selon la revendication 7, caractérisé en ce que l'on opère en présence d'hydrosulfite de sodium ou de Rongalit.
